# FASCICULE DE BREVET EUROPEEN

(11) **EP 1 765 695 B1**
(45) Date de publication et mention de la délivrance du brevet: **26.01.2011**
(21) Numéro de dépôt: 05776486.2
(22) Date de dépôt: 13.06.2005
(51) Int. Cl.: B65D 83/14, B05B 11/00, A61M 15/00

(54) **Inhalateur**
Inhalator
Inhalator

(30) Priorité: 14.06.2004 FR 0451164
(43) Date de publication de la demande: 28.03.2007
(73) Titulaire: Valois SAS, 27110 Le Neubourg (FR)
(72) Inventeur: POULARD, Fabien, 76000 ROUEN (FR)
(74) Mandataire: CAPRI
(86) Numéro de dépôt international: PCT/FR2005/050438
(87) Numéro de publication internationale: WO 2006/000730

(56) Documents cités:
- EP-A- 0 839 544
- EP-A- 1 163 922
- FR-A- 1 113 454
- GB-A- 191 111 635

## Description

La présente invention concerne un dispositif de distribution de produit fluide.

Les domaines d'application privilégiés d'un tel dispositif sont notamment mais pas exclusivement la pharmaceutique, la cosmétique et la parfumerie.

Les dispositifs de distribution de l'art antérieur comprennent généralement un réservoir de produit fluide sur lequel est monté un organe de distribution tel qu'une pompe ou une valve. Cet organe comprend généralement une chambre de dosage dans laquelle une tige d'actionnement ou soupape est montée coulissante. En particulier dans le cas de dispositifs aérosols, dans lesquels le produit est expulsé au moyen d'un gaz propulseur à travers une valve doseuse, le réservoir est généralement monté coulissant dans le corps pour actionner la soupape de valve. Le raccordement du corps avec la soupape monté sur le réservoir définit une position d'utilisation du dispositif dans laquelle ladite soupape coopère avec le corps. Dans cette position d'utilisation, la soupape est alors déplaçable entre une position de repos et une position de distribution suite à un actionnement par l'utilisateur. Les documents EP-1 163 922 et EP-0 839 544 décrivent des dispositifs de ce type. Généralement, il est nécessaire au bout de plusieurs actionnements de la soupape de nettoyer l'interface entre la soupape et le corps. En effet, des reliquats de produit fluide distribués peuvent se déposer sur les parois du manchon de raccordement coopérant avec la soupape. Or, ces reliquats sont susceptibles de polluer le produit à distribuer restant dans le réservoir voire même d'entraver la sortie de produit fluide. Pour ce nettoyage, l'utilisateur doit alors désengager la soupape du manchon de raccordement et sortir le réservoir hors du corps. Ainsi, ce désengagement donne lieu à une position de retrait du dispositif dans lequel la soupape ne coopère pas avec le corps. Le corps ainsi que le réservoir sont alors susceptibles d'être égarés ou alors, suite à une opération de nettoyage, il existe des risques d'interchanger des corps et des réservoirs appartenant à des utilisateurs différents. Cette situation peut notamment se produire dans des services de soins hospitaliers au niveau desquels les dispositifs de distribution sont nettoyés en série. Ainsi, si deux réservoirs sont interchangés, il existe des risques éventuels de problèmes hygiéniques et notamment de contaminations microbiologiques pour l'utilisateur notamment lorsque le dispositif de distribution est un dispositif de distribution par voie buccale. D'autre part, si des réservoirs ne portant pas d'informations sur l'identité du produit qu'ils contiennent sont intervertis, alors l'utilisateur dans le cas de produit pharmaceutique, peut absorber ou inhaler un produit contre-indiqué ayant des effets allergisants ou susceptibles d'engendrer des incompatibilités médicamenteuses. Le fait d'interchanger deux réservoirs après un nettoyage est particulièrement dangereux lorsque le dispositif est pourvu d'un compteur ou indicateur de doses. En effet, une erreur de réservoir pourrait avoir des conséquences dramatiques si l'utilisateur se retrouve avec un compteur affichant un nombre de doses distribué inférieur à celui restant en réalité dans le réservoir. En effet, l'utilisateur risque alors de se retrouver avec un réservoir vide alors que le compteur lui indique qu'il reste des doses à distribuer. Le risque pour la santé est alors très important.

La présente invention a pour but de fournir un inhalateur qui ne reproduit pas les inconvénients susmentionnés.

Plus particulièrement, la présente invention a pour but de fournir un inhalateur qui soit simple et peu coûteux à fabriquer et à assembler.

La présente invention a donc pour objet un inhalateur selon la revendication 1. Ainsi, le réservoir et l'organe de distribution sont en permanence reliés au corps de sorte qu'il sera impossible d'interchanger accidentellement les réservoirs après nettoyage, au moment où ils sont remis en place dans le corps.

Avantageusement, ledit réservoir est relié audit corps par des moyens de liaison déformables et/ou déplaçables permettant audit réservoir d'être inséparable dudit corps.

Selon une première variante de réalisation, lesdits moyens de liaison comprennent un cordon flexible reliant ledit corps et ledit réservoir.

Avantageusement, ledit cordon flexible est fixé au corps à proximité de ladite ouverture.

Selon une seconde variante de réalisation, lesdits moyens de liaison comprennent une tige, notamment rigide, reliant ledit corps et ledit réservoir, ladite tige comportant une première extrémité montée coulissante dans un rail de guidage ménagé sur au moins une partie de la hauteur dudit corps, et une seconde extrémité reliée audit réservoir.

Avantageusement, ladite tige est déplaçable en translation et en rotation par rapport audit corps.

Avantageusement, ledit réservoir est mobile par rapport à ladite tige.

Avantageusement, lesdits moyens de liaison comportent une bague de fixation apte à être fixée, emmanchée et/ou encliquetée sur ledit réservoir.

Avantageusement, lesdits moyens de liaison permettent un positionnement du corps et du réservoir côte à côte en position de retrait dudit réservoir libérant ainsi la totalité de la surface de l'ouverture du corps.

D'autres caractéristiques et avantages de la présente invention apparaîtront plus clairement au cours de la description détaillée suivante de deux modes de réalisation de l'invention, faite en référence aux dessins joints, donnés à titre d'exemples non limitatifs et sur lesquels :
- la figure 1 est une vue schématique de côté partiellement découpée d'un inhalateur selon un premier mode de réalisation de l'invention avec un réservoir en position d'utilisation,
- la figure 2a est une vue schématique de face de l'inhalateur de la figure 1 avec un réservoir en position de retrait,
- la figure 2b est une vue schématique de dessus de l'inhalateur de la figure 2a,
- la figure 3 est une vue schématique de derrière partiellement découpée d'un inhalateur selon un deuxième mode de réalisation de l'invention avec un réservoir en position d'utilisation,
- la figure 4a est une vue schématique de derrière de l'inhalateur de la figure 3 avec un réservoir en position de retrait, et
- la figure 4b est une vue schématique de dessus de l'inhalateur représenté en figure 4a.

L'inhalateur, selon la présente invention comporte avantageusement cinq éléments constitutifs, à savoir un réservoir 1, un organe de distribution 2, un organe de fixation 5, un corps 3 et des moyens de liaison 4.

Le réservoir 1 peut présenter toute forme appropriée. Ce réservoir comporte un fût généralement cylindrique pourvu d'une extrémité inférieure obturée par un fond et d'une extrémité supérieure à partir de laquelle un col fait saillie. Ce col définit un passage ouvert faisant communiquer l'intérieur du réservoir avec le milieu atmosphérique extérieur. Ce réservoir peut comporter du produit fluide à pression atmosphérique ou du produit fluide pressurisé.

L'organe de distribution 2 peut être une pompe ou une valve, de préférence une valve doseuse, dont le fonctionnement ne sera pas expliqué plus en détail. Dans le cas d'une valve, généralement utilisée avec des dispositifs du type aérosol, on prévoit une soupape 20 déplaçable dans une chambre de dosage pour expulser la dose du produit au moyen d'un gaz propulseur. Le réservoir 1 est monté coulissant axialement dans ledit corps. Ce déplacement axial provoque le déplacement de la soupape et donc l'actionnement de la valve permettant ainsi le vidage de ladite chambre de dosage.

L'organe de fixation 5 a pour fonction de fixer l'organe de distribution 2 sur le réservoir 3. Cet organe 5 peut être fixé sur le col du réservoir de façon connue par sertissage, encliquetage, vissage ou similaire.

Le corps 3 est selon le mode de réalisation représenté sur les différentes figures un corps typique pour inhalateur. Ce corps comporte une coque sensiblement cylindrique 34 comprenant une extrémité supérieure 31 définissant une ouverture 310 et une extrémité inférieure 33 obturée par une paroi sensiblement oblique 330. Un embout 30 pourvu d'un orifice de distribution 300 est disposé dans le prolongement de la paroi oblique 330 et fait saillie vers l'extérieur à partir de la coque 34. Un manchon de raccordement 331 fait saillie vers le haut à partir de l'extrémité inférieure 33 du corps. Il est à noter que le corps tel que décrit n'est qu'un exemple de réalisation. Des corps de tout autre configuration pourraient très bien être envisagés tels que ceux conventionnellement utilisés pour des applications nasales, otologiques ou encore dans le domaine de la parfumerie ou de la cosmétique.

Le réservoir 1 est introduit dans le corps 3 par l'ouverture 310. Ensuite, le réservoir 1 est monté en position d'utilisation sur le corps 3 par emmanchage de la tige d'actionnement 20 dans le manchon de raccordement 331. Dans le cas présent, l'actionnement de l'inhalateur représenté peut se faire de façon classique en appuyant sur le fond du réservoir. Dans la position dite d'utilisation du réservoir, la valve 2 coopère avec le corps 3 entre une position de repos et une position de distribution. La position de repos correspond à la position de la soupape 20 dans le manchon 331 lorsque la valve 2 n'est pas sollicitée par un actionnement de l'inhalateur par l'utilisateur. La position de distribution correspond à la position de la soupape 20 dans le manchon 331 lors d'un actionnement de l'inhalateur par l'utilisateur. Le réservoir 1 définit également une position de retrait lorsque la soupape 20 est désengagée du manchon de raccordement 331 et le réservoir retiré du corps 3. Dans ce cas, l'organe de distribution 2 ne coopère plus avec le corps 3 et ne peut donc plus être actionné par l'utilisateur.

Selon l'invention, des moyens de liaison 4, 4' sont prévus pour solidariser en toutes positions le réservoir 1 et le corps 3. Dans ce cas, le terme « réservoir » englobe à la fois le réservoir, l'organe de distribution ainsi que l'organe de fixation qui sont comme précédemment vu assemblés ensemble. Ainsi, le réservoir 1, l'organe de distribution 2 et l'organe de fixation 5 pourront être en permanence reliés au corps 3, même en position de retrait dudit organe de distribution 2.

Deux variantes de réalisation des moyens de liaison 4, 4' peuvent par exemple être envisagées.

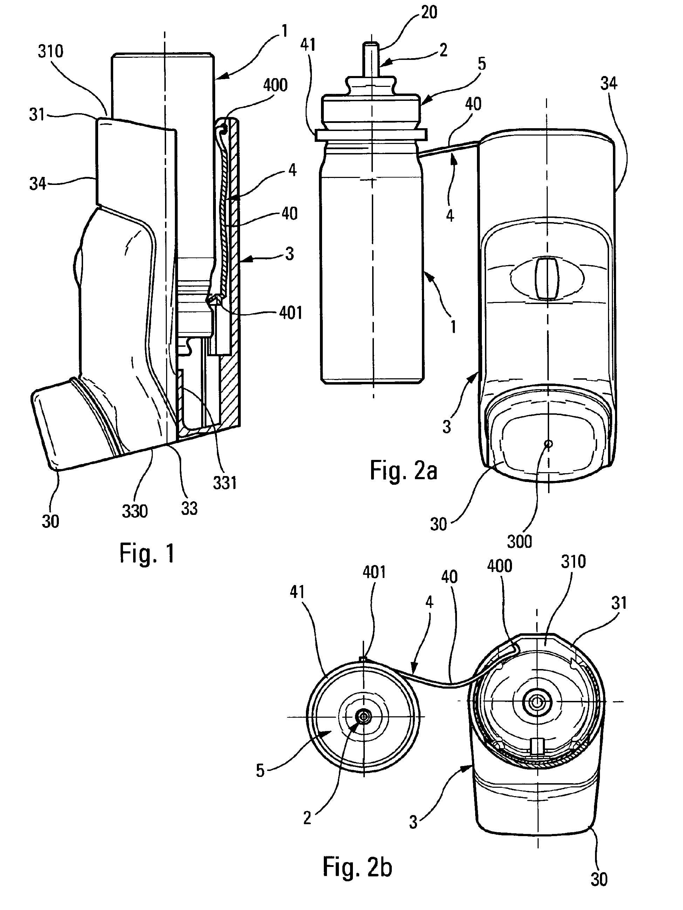

Selon une première variante de réalisation représentée sur les figures 1, 2a et 2b, les moyens de liaison 4 comprennent un cordon flexible 40. Ce cordon présente un certain degré de déformabilité ou d'élasticité de sorte à pouvoir facilement se plier et/ou s'étirer. Ce cordon comprend une première extrémité 400 et une seconde extrémité 401. Ces deux extrémités 400, 401 sont reliées respectivement au corps 3 et au réservoir 1. Ces extrémités 400, 401 peuvent être réalisées monobloc avec le corps et/ou le réservoir ou bien être des pièces rapportées fixées par tout moyen approprié au niveau d'une surface ou d'un logement correspondant. Avantageusement, l'extrémité 400 est fixée au niveau de la coque 34 à proximité de l'ouverture 310. L'extrémité 401 est quant à elle avantageusement reliée à une bague de fixation 41 apte à être fixée, emmanchée et/ou encliquetée sur le réservoir 1, tel que visible particulièrement sur la figure 2b. Dans ce cas, la bague peut 41 se loger sous l'organe de fixation 5 et est ainsi être confinée dans un espace localisé entre le réservoir 1 et l'organe de fixation 5.

Pour passer d'une position d'utilisation représentée sur la figure 1 à une position de retrait représentée sur les figures 2a et 2b, l'utilisateur exerce une traction sur le réservoir pour désengager la tige 20 du manchon 331. La flexibilité du cordon 40 permet alors une extraction du réservoir 1 du corps 3 et libère ainsi l'accès à l'intérieur du corps 3 en dégageant totalement la surface de l'ouverture 310 de ce corps. Le réservoir 1 et le corps 3, mobiles l'un par rapport à l'autre, peuvent alors être positionnés côte à côte afin de ne pas gêner l'accès au manchon 331 à nettoyer.

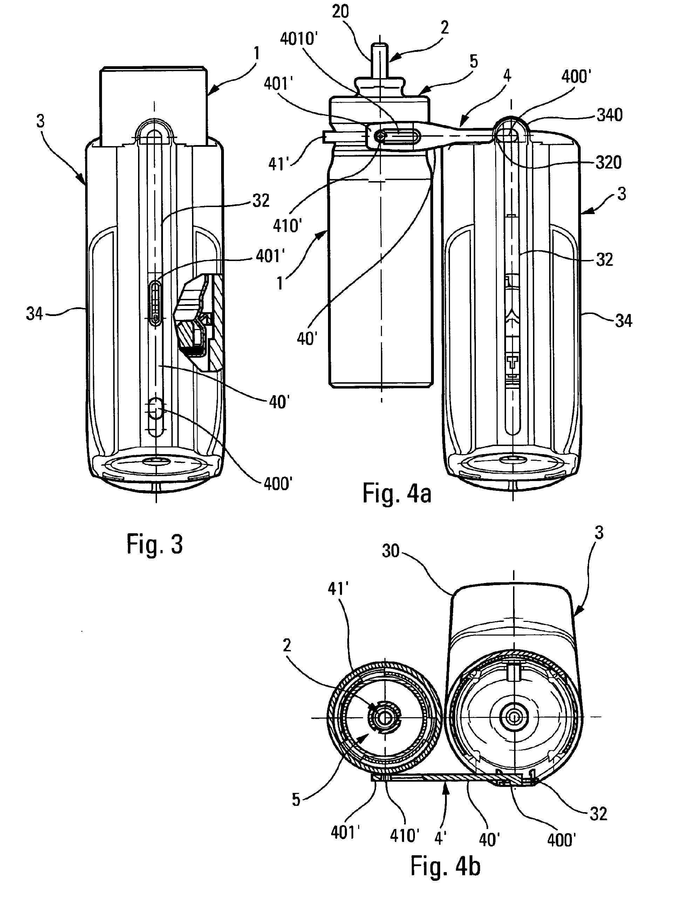

Selon une deuxième variante de réalisation représentée sur les figures 3, 4a et 4b, les moyens de liaison 4' comprennent une tige ou biellette 40', de préférence rigide, comportant une première extrémité 400' et une seconde extrémité 401'. Ces deux extrémités 400', 401' sont respectivement reliées au corps 3 et au réservoir 1. La première extrémité 400' est montée coulissante dans une sorte de rail de guidage 32 ménagé sur au moins une partie de la hauteur du corps 3, de façon à permettre un déplacement vertical du réservoir par rapport au corps. La seconde extrémité 401' comporte quant à elle selon un mode de réalisation particulier, un alésage longitudinal 4010' dans lequel est monté coulissant un élément de liaison 410' relié à une bague de fixation 41' fixée au réservoir. La bague de fixation 41' peut comme dans la variante de réalisation précédente être logée dans un espace situé ente l'organe de fixation 5 et le réservoir 1. De façon similaire, cette bague peut être fixée, emmanchée, encliquetée ou similaire sur ledit réservoir. Il est à noter que l'élément de liaison 410' peut être assemblé sur la bague de fixation 41' de façon à être détachable, par exemple par dévissage.

Pour passer d'une position d'utilisation représentée sur la figure 3 à une position de retrait représentée sur les figures 4a et 4b, l'utilisateur doit exercer une traction sur le réservoir 1 afin de déplacer le réservoir 1 par rapport au corps 3 en faisant coulisser la première extrémité 400' de la biellette ou tige 40' dans le rail de guidage 32. En fin de coulissement, la première extrémité 400' atteint un anneau de rotation 340 apte à faire pivoter la tige 40', par exemple de 90°. Cette rotation peut être limitée par un bord transversal adjacent 320 du rail 32. Ce bord transversal peut par exemple correspondre à une partie de l'extrémité supérieure du corps 3, tel que visible notamment sur la figure 4a. Cette rotation permet ainsi de dégager l'accès à l'intérieur du corps 3 et de libérer ainsi la surface de l'ouverture 310 du corps 3. Ensuite, l'élément de liaison 410' peut être déplacé au niveau de l'alésage 4010' afin de décaler le réservoir 1 par rapport au corps 3 et ainsi permettre au réservoir 1 de pivoter, par exemple d'approximativement 180°, pour venir se positionner côte à côte avec le corps 3. Ainsi, ni les moyens de liaison 4, ni le réservoir 1 n'entravent l'accès au corps 3 et notamment au manchon de raccordement 331 à nettoyer.

Selon un mode de réalisation particulièrement avantageux, le corps 3 peut être muni d'un dispositif de comptage ou d'indication des doses de produit fluide distribuées ou restant à distribuer à partir du réservoir 1. Par exemple, ce dispositif de comptage ou d'indication peut être actionné par le déplacement du réservoir entre ses positions de repos et de distribution. Grâce à la présence des moyens de liaison 4, 4', l'utilisateur ou le préposé au nettoyage ne peut pas interchanger accidentellement les réservoirs après un nettoyage. Par conséquent, le réservoir sera toujours forcément associé à son corps et le nombre de doses indiqué au niveau du corps correspondra forcément au bon nombre de doses distribuées ou restant à distribuer dans le réservoir.

## Revendications

1. Inhalateur de produit fluide pharmaceutique comprenant :
- un réservoir (1) de produit fluide pharmaceutique à distribuer,
- un organe de distribution (2), tel qu'une pompe ou une valve, monté sur ledit réservoir (1), et
- un corps (3) apte à recevoir ledit réservoir (1), ledit corps (3) étant pourvu d'un orifice de distribution et d'une ouverture (310) par laquelle ledit réservoir (1) peut être introduit dans le corps (3), ledit réservoir (1) étant déplaçable entre une position d'utilisation, dans laquelle ledit organe de distribution (2) coopère avec ledit corps (3) entre une position de repos et une position de distribution, et une position de retrait dans laquelle ledit organe de distribution (2) ne coopère pas avec ledit corps (3), ledit corps (3) étant muni d'un dispositif de comptage ou d'indication des doses de produit fluide distribuées ou restant à distribuer à partir du réservoir, ledit dispositif d'indication ou de comptage étant actionné par le déplacement du réservoir entre ses positions de repos et de distribution, **caractérisé en ce que** ledit réservoir (1) est en toutes positions solidarisé audit corps (3).

2. Inhalateur selon la revendication 1, dans lequel ledit réservoir (1) est relié audit corps (3) par des moyens de liaison (4, 4') déformables et/ou déplaçables permettant audit réservoir (1) d'être inséparable dudit corps (3).

3. Inhalateur selon la revendication 2, dans lequel lesdits moyens de liaison (4) comprennent un cordon flexible (40) reliant ledit corps (3) et ledit réservoir (1).

4. Inhalateur selon la revendication 3, dans lequel ledit cordon flexible (40) est fixé au corps (3) à proximité de ladite ouverture (31).

5. Inhalateur selon la revendication 2, dans lequel lesdits moyens de liaison (4') comprennent une tige (40'), notamment rigide, reliant ledit corps (3) et ledit réservoir (1), ladite tige (40') comportant une première extrémité (400') montée coulissante dans un rail de guidage (32) ménagé sur au moins une partie de la hauteur dudit corps (3), et une seconde extrémité (401') reliée audit réservoir (1).

6. Inhalateur selon la revendication 5, dans lequel ladite tige (40') est déplaçable en translation et en rotation par rapport audit corps (3).

7. Inhalateur selon la revendication 5 ou 6, dans lequel ledit réservoir (1) est mobile par rapport à ladite tige (40').

8. Inhalateur selon l'une quelconque des revendications 2 à 7, dans lequel lesdits moyens de liaison (4, 4') comportent une bague de fixation (41, 41') apte à être fixée, emmanchée et/ou encliquetée sur ledit réservoir (1).

9. Inhalateur selon la revendication 2, dans lequel lesdits moyens de liaison (4, 4') permettent un positionnement du corps (3) et du réservoir (1) côte à côte en position de retrait dudit réservoir (1) libérant ainsi la totalité de la surface de l'ouverture du corps (3).

## Claims

1. An inhaler for pharmaceutical fluid comprising:
• a reservoir (1) of pharmaceutical fluid to be dispensed;
• a dispenser member (2), such as a pump or a valve, mounted on said reservoir (1); and
• a body (3) that is suitable for receiving said reservoir (1), said body (3) being provided with a dispenser orifice and with an opening (310) via which said reservoir (1) can be inserted into the body (3), said reservoir (1) being displaceable between a working position, in which said dispenser member (2) cooperates with said body (3) between a rest position and a dispensing position, and a withdrawn position in which said dispenser member (2) does not co-operate with said body (3), said body (3) being provided with a counter or indicator device for counting or indicating the number of doses of fluid that have been dispensed or that remain to be dispensed from the reservoir, said indicator or counter device being actuated by the reservoir being displaced between its rest and dispensing positions, the device being **characterized in that** said reservoir (1) is secured to said body (3) in all positions.

2. An inhaler according to claim 1, in which said reservoir (1) is connected to said body (3) by deformable and/or displaceable connection means (4, 4'), enabling said reservoir (1) to be inseparable from said body (3).

3. An inhaler according to claim 2, in which said connection means (4) comprise a flexible cord (40) connecting said body (3) and said reservoir (1).

4. An inhaler according to claim 3, in which said flexible cord (40) is fastened to the body (3) in the proximity of said opening (31).

5. An inhaler according to claim 2, in which said connection means (4') comprise a rod (40'), in particular a rigid rod, connecting said body (3) and said reservoir (1), said rod (40') including a first end (400') that is slidably mounted in a guide rail (32) formed over at least a fraction of the height of said body (3), and a second end (401') connected to said reservoir (1).

6. An inhaler according to claim 5, in which said rod (40') is displaceable in translation and in rotation relative to said body (3).

7. An inhaler according to claim 5 or claim 6, in which said reservoir (1) is movable relative to said rod (40').

8. An inhaler according to any one of claims 2 to 7, in which said connection means (4, 4') include a fastener ring (41, 41') that is suitable for being fastened, engaged, and/or snap-fastened on said reservoir (1).

9. An inhaler according to claim 2, in which said connection means (4, 4') enable the body (3) and the reservoir (1) to be positioned side by side, when said reservoir (1) is in its withdrawn position, thereby releasing the entire surface of the opening of the body (3) .

## Patentansprüche

1. Inhalator für ein pharmazeutisches Fluidprodukt ausweisend:
- einen Vorratsbehälter (1) für ein abzugebendes pharmazeutisches Fluidprodukt,
- ein auf dem Vorratsbehälter (1) angebrachtes Abgabeorgan (2), wie etwa eine Pumpe oder ein Ventil, und
- einen Körper (3) zur Aufnahme des Vorratsbehälters (1), wobei der Körper (3) mit einer Abgabeöffnung und einer Öffnung (310) versehen ist, durch die der Vorratsbehälter (1) in den Körper einführbar ist, wobei der Vorratsbehälter (1) zwischen einer Nutzungsstellung, in der das Abgabeorgan mit dem Körper (3) zusammenwirkt, zwischen einer Ruhestellung und einer Rückzugstellung verschiebbar ist, in der das Abgabeorgan (2) nicht mit dem Körper (3) zusammenwirkt, wobei der Körper (3) mit einer Vorrichtung zum Zählen und Anzeigen von Fluidproduktdosen versehen ist, die von dem Vorratsbehälter ausgegeben werden oder zum Ausgeben dort verbleiben, wobei die Zähl- und Anzeigevorrichtung durch Verschieben des Vorratsbehälters zwischen den Ruhe- und Abgabestellungen betätigt wird, **dadurch gekennzeichnet, dass** der Vorratsbehälter (1) in sämtlichen Stellungen mit dem Körper (3) fest verbunden ist.

2. Inhalator nach Anspruch 1, wobei der Vorratsbehälter (1) mit dem Körper (3) durch verformbare und/oder verschiebbare Verbindungsmittel (4, 4') verbunden ist, wodurch der Vorratsbehälter (1) vom Körper (3) untrennbar ist.

3. Inhalator nach Anspruch 2, wobei die Verbindungsmittel (4) eine flexible Kordel (40) umfassen, die den Körper (3) mit dem Vorratsbehälter (1) verbindet.

4. Inhalator nach Anspruch 3, wobei die flexible Kordel (40) am Körper (3) nahe der Öffnung (31) fest angebracht ist.

5. Inhalator nach Anspruch 2, wobei die Verbindungsmittel (4') eine insbesondere starre Stange (40') umfassen, die den Körper (3) mit dem Vorratsbehälter (1) verbindet, wobei die Stange (40') ein erstes Ende (400') aufweist, das in einer Führungsschiene (32) gleitend angebracht ist, die auf zumindest einem Teil der Höhe des Körpers (3) ausgebildet ist, und ein zweites Ende (401'), das mit dem Vorratsbehälter (1) verbunden ist.

6. Inhalator nach Anspruch 5, wobei die Stange (40') translationsmäßig und drehmäßig relativ zum Körper (3) verschiebbar ist.

7. Inhalator nach Anspruch 5 oder 6, wobei der Vorratsbehälter (1) relativ zur Stange (40) beweglich ist.

8. Inhalator nach einem der Ansprüche 2 bis 7, wobei die Verbindungsmittel (4, 4') einen Festlegungsring (41, 41') umfassen, der dazu bestimmt ist, auf dem Vorratsbehälter (1) aufgedrückt und/oder verrastet festgelegt zu werden.

9. Inhalator nach Anspruch 2, wobei die Verbindungsmittel (4, 4') eine Positionierung des Körpers (3) und des Vorratsbehälters (1) nebeneinander in der Rückzugstellung des Vorratsbehälters (1) unter Freigabe der gesamten Oberfläche der Öffnung des Körpers (3) erlaubt.
